# EUROPEAN PATENT SPECIFICATION

(11) **EP 0 896 547 B1**
(45) Date of publication and mention of the grant of the patent: **26.06.2002**
(21) Application number: 97922232.0
(22) Date of filing: 18.04.1997
(51) Int. Cl.: A61K 49/00

(54) **DIAGNOSTIC COMPOSITION**
DIAGNOSTISCHE ZUSAMMENSETZUNG
COMPOSITION A USAGE DIAGNOSTIQUE

(30) Priority: 30.04.1996 SE 9601659
(43) Date of publication of application: 17.02.1999
(73) Proprietor: Diabact AB, 751 05 Uppsala (SE)
(72) Inventor: NYSTRÖM, Christer, S-756 61 Uppsala (SE); PETTERSSON, Anders, S-442 97 Kode (SE); LUNDQVIST, Thomas, S-752 63 Uppsala (SE)
(74) Representative: Onn, Thorsten
(86) International application number: SE9700659
(87) International publication number: WO9740856

(56) References cited:
- WO-A-86/06625
- WO-A-95/11672
- WO-A-96/14091
- US-A- 4 947 861

## Description

Gastric ulcer disorders constitute a serious health problem and cost communities large sums of money in the form of medical costs and absenteeism. It has been fully established that the presence of *Helicobacter pylori* in the stomach is a necessary prerequisite for the development of gastric stomach ulcers and/or duodenum ulcers. If the stomach contains no *Helicobacter pylori*, then no gastric ulcers will develop. It has also been found that there will be no reoccurrence of the gastric ulcer disorder, when the *Helicobacter pylori* infection has been cured with the aid of antibiotics. Thus, gastric ulcer disorders are today considered to be disorders that are caused by bacteria and that can, and shall, be cured.

There is a great need for a reliable and simple method of diagnosis, since large patient populations suffering from gastrointestinal problems will need to be examined for the presence of *Helicobacter pylori* infections.

The present invention is based on the knowledge that the bacterium *Helicobacter pylori* produces the enzyme urease in very large quantities. The enzyme urease is not normally found in human beings and the presence of the enzyme in the human stomach is a clear indication that *Helicobacter pylori* bacteria are also present. *Helicobacter pylori* have established an ecological niche in the human stomach. The bacterium thrives in a neutral pH, which is found beneath the mucosal layer of the stomach. It produces large quantities of the enzyme urease, which, in turn, catalyzes the decomposition of urea to ammonia and bicarbonate. This bicarbonate is then converted in the acid environment of the stomach into carbon dioxide and water.

In Sweden, about 470,000 patients seek medical care annually for ailments and disorders resembling or relating to gastric ulcers. It is thus of paramount interest to be able to diagnose and treat *Helicobacter pylori* infections in a simple and reliable manner, both with respect to health economy and with respect to the patients concerned.

The present invention relates to a solid preparation which enables the ongoing urease activity in the stomach in conjunction with *Helicobacter pylori* infection to be diagnosed both simply and reliably.

### Present-day methods available for demonstrating the presence of Helicobacter pylori

### Biopsy methods

A common feature of these methods (bacteria cultivation, histologic examinations, fast urea tests) is that they are all performed on biopsy material. This involves subjecting the patient to a gastroscopic examination of the stomach with the aid of fibre optics, during which samples of tissue from the stomach mucus membrane are taken out. This is both an expensive procedure and one which is unpleasant to the patient concerned.

### Serological methods

A common feature of these methods involves determining the presence of specific antibodies against *Helicobacter pylori in* the blood or in the stomach secretion. One drawback of these methods is that it is necessary to take blood samples or to take secretion from the stomach. Another drawback with serological methods is that it cannot be shown that bacteria are present at the time of performing the examination.
- It takes about twenty days from the time of being infected with *Helicobacter pylori* to the time at which antibodies against the bacterium can be shown to exist.
- After the bacterial infection has been successfully treated, elevated concentrations of antibodies remain in the blood for a very long period of time, therewith greatly limiting the possibility of performing an accurate follow-up examination.

### Urea-exhalation test

This method is based on the production of the enzyme urease by the bacteria *Helicobacter pylori.* Urease catalyzes the decomposition of urea to ammonia and bicarbonate. The bicarbonate is then converted to carbon dioxide and water in the acidic environment of the stomach.

The urea-exhalation test is, at present, carried out in the following way: The patient swallows an aqueous solution that contains isotope labelled (¹¹C, ¹³C, ¹⁴C) urea. When the stomach contains urease-producing *Helicobacter pylori* and acid, urea is broken down and the isotope-labelled carbon atoms convert to carbon dioxide which is secreted via the exhaled air. The concentration of isotope-labelled carbon dioxide in the exhaled air is then determined.

In another variant, the concentration of the other decomposition product, namely ammonia, is determined instead. The carbon in the urea need not be isotope-labelled in this latter case. The nitrogen may be isotope-labelled, although not necessarily so, since ammonia is not normally present in exhalation air. The ammonia that is formed passes through the wall of the stomach and into the blood circulation, and then passes the liver and is secreted in the exhaled air over the pulmonary alveoles.

The advantages with this method is that the examination is non-invasive, i.e. the patient need not be subjected to the discomfort of instruments (gastroscopy), and no blood samples or samples of stomach secretion need be taken. Furthermore, the fact that the bacterium has a very local propagation area has no significance, since the urea administered will spread over the entire surface of the stomach.

Swedish Patent Application 9403755-3 filed on November 2, 1994 and corresponding to International Patent Application PCT/SE95/01212 (WO96/14091) describes diagnostic medical preparation which contains isotope-labelled urea, which is essentially water-free and which preferably contains a solid, water-soluble acid. The preparation is used to demonstrate the presence of *Helicobacter pylori* in the stomach, by determining the presence in exhalation air of the isotope-labelled carbon dioxide formed by the urease of the bacterium. This publication makes no mention of demonstrating the presence of ammonia in expiration air.

U.S. Patent Specification US-A 4,947,861 describes how the presence of *Campylobacter pylori* in the stomach can be demonstrated by determining the presence in expiration air of ammonia that is generated by the action of the urease formed by the bacterium on urea. This publication merely mentions that the patient shall ingest urea, but does not mention the form in which the urea shall be ingested. Furthermore, it takes from ten to sixty minutes after having ingested the urea before a test result can be obtained (column 3, lines 52-53). In distinction, it takes at most ten minutes to obtain a test result when ingesting the water-free preparation of the present invention.

The disadvantages with urea-exhalation tests are related to a number of facts:
1. The urea is ingested as an aqueous solution. This enables urea to be broken down by urease-producing bacteria in the oral cavity and pharynx of the patient, which can result in a falsely positive result with regard to the presence of *Helicobacter pylori.*
2. In order for the test to function correctly when analyzing carbon dioxide, it is necessary for a given quantity of hydrogen ions to be present in the stomach in order for the bicarbonate formed to be broken down to carbon dioxide. Many patients suffering from gastric ulcer disorders are treated with acid-secretion inhibiting drugs which cause falsely negative results when examining such patients with present-day test methodology.
3. Urea has a short chemical stability in aqueous solution, which presents practical difficulties when applying the test in clinical practices. Furthermore, isotope-labelled urea is relatively expensive.
4. The use of isotope-labelled urea requires complicated and expensive mass spectrometer type apparatus to show a specific carbon isotope. The isotope ¹⁴C is, however, radioactive and can be shown more easily by scintillation measuring processes.

However, the ingestion of radioactive material by the patient is not to be recommended, even though the ingested dosage is small.

It will be evident from the aforegoing that several diagnostic methods for demonstrating the presence of *Helicobacter pylori* in the stomach are known and that these methods are encumbered with a number of drawbacks, such as the inability to show positively the presence of bacteria, besides being unpleasant to the patient.

There is thus a great need for improved diagnostic methods which will provide greater diagnostic reliability under all conditions, with enhanced practical use.

### General description of the invention

The formulation of an essentially water-free capsule, tablet or some other highly dissolvable solid preparation that contains urea provides several significant advantages with regard to urea-exhalation tests carried out to determine the presence of ammonia in the diagnosis of gastric ulcer disorders:
1. The administration of urea in a solid preparation prevents urease-producing bacteria in the oral cavity and pharynx from breaking down urea before it has reached the stomach. This significantly increases the reliability of the examination, since it reduces the number of falsely positive results.
2. Analyzing with respect to the presence of ammonia also enables the urea-exhalation test to be used during an ongoing acid-secretion inhibiting treatment, since the formation of ammonia does not require an elevated hydrogen-ion concentration as distinct from the formation of carbon dioxide.
   This brings about a significant gain, since the patients subjected to the urea-exhalation test are treated chronically with potent acid-secretion inhibiting drugs, therewith significantly reducing the occurrence of falsely negative results.
3. Because water-free urea in a capsule, tablet or some other solid preparation is stable over a longer time period, the problems associated with the limited chemical stability of urea in aqueous solution are avoided.
   This provides significant practical advantages with respect to the distribution and the use of the urea-breath test.
4. The use of a solid preparation (e.g. a tablet) also has dosage accuracy and time-saving advantages in comparison with preparing the established water-based test liquid.
5. When demonstrating the presence of *Helicobacter pylori* by determining the concentration of formed carbon dioxide, it is necessary to use expensive isotope-labelled urea and expensive and complicated mass spectrometry or radiological apparatus to distinguish the carbon dioxide that is formed by the bacteria from the carbon dioxide that is normally formed by the metabolism of the organism. This is not necessary when demonstrating the presence of formed ammonia, since ammonia is not normally formed in the metabolism of the organism.

In order to be able to achieve these significant advantages, it is necessary to construct the solid preparation such that its contents will not be influenced by urease in the oral cavity and pharynx of the patient, and such that it will decompose very rapidly and completely in the stomach.

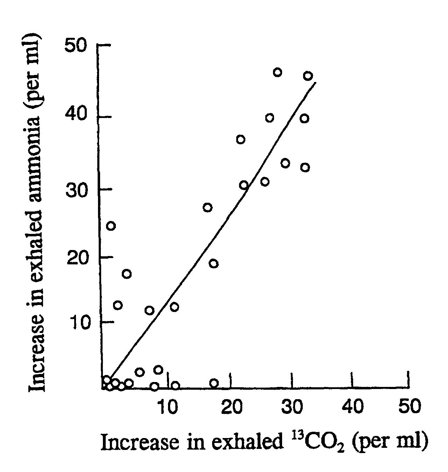

The accompanying drawing shows the results obtained with a comparison test carried out with a preparation of the invention in accordance with the following Example.

### Technical description of the invention

In accordance with the invention, there is provided a solid preparation which contains primarily urea which is free from labelled isotopes, and substantially no water, said preparation being intended for peroral administration to humans.

In one suitable embodiment, the preparation also contains a solid, water-soluble base, such as sodium bicarbonate. The addition of a base facilitates that the ammonia is transferred into the blood and from there to the lungs and does not react with stomach acid to form a salt. In one preferred embodiment, the base is added in an amount at which pH in the stomach will be in the range of from 4 to 8, particularly in the proximity of the preparation.

The pharmaceutical preparation of the invention is composed such that urea will be released essentially instantaneously when the preparation reaches the stomach. The preparation may either be in conventional tablet form, optionally provided with a quickly soluble coating, although it may also be in capsule form. Primarily so-called hard gelatine capsules (operculate capsules) are contemplated, although soft gelatine capsules may also be used. The only condition in this respect is that the chemical stability will be sufficiently high and that the active substance content of the preparation will be released and dissolve quickly. The chemical stability is, as a rule, improved when the active substance and excipients exist in a solid form. If an oil solution is used in combination with a soft gelatine capsule, the stability will normally be sufficiently high.

Other solid preparations may also be used. The use of solid solutions, solid dispersions and pellet preparations can be mentioned by way of example, without limiting the list of possible preparations. Pellet preparations include larger or smaller grains produced by granulation or extrusion/spheronization. The active substance can then be incorporated in the pellets, or affixed to the surfaces thereof. The active pellets can then be compressed to form tablets that will disintegrate quickly, or the pellets can be encased in hard gelatine capsules.

With regard to tablets, which are the preferred embodiment of the preparation of the invention, the tablets can be produced with the aid of excipients and process steps that are well known to the person skilled in this art. Thus, in order to obtain maximum quick release and dissolution, there will preferably be used essentially water-soluble components, and excipients that accelerate the disintegration of the tablet. The excipient used may be a pharmaceutical disintegrating or bursting agent of the kind known to the skilled person. Those agents that swell markedly in water as a result of hydration and increase in volume by 10-20 times their dry volume are particularly suitable in this regard. Examples of such agents are cellulose and starch derivatives in the form of crosslinked polymers which while being insoluble in water swell markedly therein. Derivatives of polyvinyl pyrrolidone is an example of such agents. Particular mention can be made to suitable pharmaceutical bursting agents that comprise a modified cellulosegum which has high swellability in water and which is commercialized under the registered trademark Ac-Di-Sol® by FMC Corporation, U.S.A. Other types of pharmaceutical disintegrating agents may also be used, and the person skilled in this art will have no problem in selecting a suitable bursting agent.

In one particularly preferred form, the active substance urea is used in a very fine particle form. In this form, the particle size is preferably below 200 µm, and more preferably below 20 µm. However, this does not exclude the use of coarser active substance particles when formulation of the preparation in other respects so permits. Such judgements and assessments are well within the expertise of the skilled person.

Several methods of determining the presence of ammonia in gas mixtures, in the present case in exhalation air, are known to the skilled person. The method described in the earlier mentioned patent specification US-A 4,947,861 may be used in this regard.

### Example

With the intention of testing and comparing the invention with conventional techniques (aqueous solution and determining the carbon dioxide concentration in exhalation air) tablets were prepared in accordance with the following:

The ingredients of the following composition were mixed in a conventional rotary mixer. Tablets were then compressed in a so-called excenter machine equipped with 12 mm cupped punches.

### Composition of one tablet

| | | |
|---|---|---|
| 1. | Urea | 450 mg |
| 2. | Mannitol | 63 mg |
| 3. | Avicel® Ph 101 | 60 mg |
| 4. | Ac-Di-Sol® | 24 mg |
| 5. | Magnesium stearate | 3 mg |

Mannitol was used conveniently in a granulated form for so-called direct compression.

Avicel® Ph 101 is the trade name of a microcrystalline cellulose retailed by FMC Corporation, U.S.A.

Ac-Di-Sol® is the trade name of a modified cellulose rubber that swells greatly in water, sold by FMC Corporation, U.S.A. It functions in this case as a pharmaceutical bursting agent for rapid disintegration of the tablet.

Patients were treated either by administering two tablets (corresponding to 900 mg urea) in accordance with the above, or with 2.5 ml of an aqueous solution containing 100 mg of ¹³C labelled urea.

In a comparison test carried out between the typical administration of an isotope-labelled urea (aqueous solution and determining carbon dioxide content) and a preparation of the invention, it was found that of the ten negative patients included in the test the presence of isotope-labelled carbon dioxide was shown to exist in the exhalation air of five patients who lacked bacteria in the stomach, ten minutes after ingesting the aqueous solution .

No ammonia was found in the exhalation air of patients who ingested urea in the form of a preparation according to the invention.

A comparison between isotope-labelled urea in an aqueous solution and in a solid preparation in accordance with the invention administered to seventeen patients for which it was known that bacteria were present in the stomach showed markedly elevated quantities of ammonia in the exhalation air ten minutes after ingestion of the tablet. In the case of those patients administered with the aqueous solution, it was necessary to wait a further ten minutes with respect to three of the patients before it could be said positively that the quantities of isotope-labelled carbon dioxide detected originated from stomach urease.

**Table 1:**

| A compilation of the results of diagnosing *Helicobacter pylori* with the aid of isotope-labelled urea in aqueous solution and in a solid preparation in accordance with the invention, respectively. | | | | | |
|---|---|---|---|---|---|
| Method | Falsely positive | | Falsely negative | | No. of patients |
| | 10 min. | 20 min. | 10 min. | 20 min. | |
| Solution - carbon dioxide | 5 | 0 | - | - | 27 |
| Tablet - ammonia | | 0 | 0 | - | 27 |

The result of the examination is also shown in the accompanying drawing. The drawing is a diagram showing the correlation between the test results obtained with conventional exhalation tests with ¹³C urea according to European standard protocol and the test results obtained with the inventive preparation of the invention. Each point in the diagram denotes an individual patient tested at short intervals by the conventional method with urea in solution and determining the carbon dioxide content of the exhalation air, and with a water-free preparation in accordance with the invention and determining the ammonia concentration in exhalation air. Samples were taken ten minutes after administration in both cases. Thus, all points should ideally lie on the 45-degree line.

Those points that lie beneath the 45-degree line denote more or less positively deviating test values obtained with the conventional solution method, and it is evident that a substantial number of falsely positive test values were obtained (the points close to the X-axis), wherein the preparation of the invention, on the other hand, gave correct zero results. It also shows that more reliable test results are obtained when using the preparation of the invention than when using the conventional solution. The analysis is also much simpler, since no mass spectrographic detection of a particular carbon isotope is required. This also greatly reduces the costs involved.

Consequently, the use of a water-free preparation in accordance with the invention in demonstrating the presence of *Helicobacter pylori* in the stomach affords a number of important advantages in comparison with the conventional solution used in accordance with earlier known techniques, these advantages not being obvious to the person skilled in this art.

## Claims

1. A medical preparation for demonstrating the presence of urease activity in the stomach in conjunction with a *Helicobacter pylori* infection, by demonstrating the presence of ormed ammonia in exhaled air, **characterized in that** said preparation is comprised of a solid, substantially water-free composition that contains urea which is influenced by the activity of *Helicobacter pylori* to form ammonia, said urea being free from labelled isotopes, and that the preparation is formulated for a rapid dissolution in the stomach subsequent to administration.

2. A preparation according to claim 1, **characterized in that** said preparation contains a solid, water-soluble base.

3. A preparation according to claim 2, **characterized in that** the solid, water-soluble base is present in such an amount that the pH in the stomach will be at lowest 4 and at highest 8, particularly in the vicinity of said preparation.

4. A preparation according to any one of claims 1-3, **characterized in that** the urea has a particle size substantially below 200 µm, and preferably then below 20 µm.

## Patentansprüche

1. Medizinisches Präparat zum Nachweis der Gegenwart von Urease-Aktivität im Magen in Verbindung mit einer *Helicobacter pylori* Infektion durch Nachweisen der Gegenwart von gebildetem Ammoniak in der ausgeatmeten Luft, **dadurch gekennzeichnet, dass** das Präparat aus einer festen, im Wesentlichen wasserfreien Zusammensetzung besteht, die Harnstoff enthält, welcher durch die Aktivität von *Helicobacter pylori zu* Ammoniak umgewandelt wird, wobei der Ammoniak frei von markierten Isotopen ist, und das Präparat für eine rasche Auflösung im Magen nach der Verabreichung formuliert ist.

2. Präparat nach Anspruch 1, **dadurch gekennzeichnet, dass** das Präparat eine feste wasserlösliche Base enthält.

3. Präparat nach Anspruch 2, **dadurch gekennzeichnet, dass** die feste wasserlösliche Base in einer solchen Menge vorliegt, dass der pH im Magen mindestens 4 und höchstens 8 beträgt, insbesondere in der Umgebung des Präparats.

4. Präparat nach einem der Ansprüche 1 bis 3, **dadurch gekennzeichnet, dass** der Harnstoff eine Partikelgrösse von im Wesentlichen kleiner als 200 µm und vorzugsweise kleiner als 20 µm aufweist.

## Revendications

1. Préparation médicale pour mettre en évidence l'existence d'une activité uréase dans l'estomac en rapport avec une infection par *Helicobacter pylori*, en démontrant la présence d'ammoniac formé dans de l'air expiré, **caractérisée en ce que** ladite préparation est constituée d'une composition solide sensiblement anhydre qui contient de l'urée, laquelle est influencée par l'activité de *Helicobacter pylori* et forme alors de l'ammoniac, ladite urée étant exempte d'isotopes marqués, et **en ce que** la préparation est formulée pour se dissoudre rapidement dans l'estomac après administration.

2. Préparation selon la revendication 1, **caractérisée en ce que** ladite préparation contient une base solide hydrosoluble.

3. Préparation selon la revendication 2, **caractérisée en ce que** la base solide hydrosoluble est présente en quantité telle que le pH dans l'estomac sera au minimum de 4 et au maximum de 8, en particulier au voisinage de ladite préparation.

4. Préparation selon l'une quelconque des revendications 1 à 3, **caractérisée en ce que** l'urée présente une taille de particules sensiblement inférieure à 200 µm et de préférence inférieure à 20 µm.
